(19) Europäisches Patentamt
European Patent Office
Office européen des brevets

(11) Veröffentlichungsnummer : **0 050 093**
**B1**

(12) # EUROPÄISCHE PATENTSCHRIFT

(45) Veröffentlichungstag der Patentschrift : **19.12.84**

(51) Int. Cl.³ : **C 07 C 69/74**, C 07 C 43/295, A 01 N 53/00

(21) Anmeldenummer : **81810335.0**

(22) Anmeldetag : **17.08.81**

(54) **Cyclopropancarbonsäure-alpha-allenyl-3-phenoxy-benzylester, ihre Herstellung und Verwendung in der Schädlingsbekämpfung.**

(30) Priorität : **22.08.80 CH 6353/80**
**30.06.81 CH 4304/81**

(43) Veröffentlichungstag der Anmeldung : **21.04.82 Patentblatt 82/16**

(45) Bekanntmachung des Hinweises auf die Patenterteilung : **19.12.84 Patentblatt 84/51**

(84) Benannte Vertragsstaaten : **AT BE CH DE FR GB IT LI NL**

(56) Entgegenhaltungen :
**EP-A- 0 001 824**
**EP-A- 0 008 333**
**DE-A- 3 225 130**
**DE-A- 3 229 661**
**US-A- 3 932 530**

(73) Patentinhaber : **CIBA-GEIGY AG**
**Postfach**
**CH-4002 Basel (CH)**

(72) Erfinder : **Ackermann, Peter, Dr.**
**Reichensteinerstrasse 12**
**CH-4153 Reinach (CH)**
Erfinder : **Gsell, Laurenz, Dr.**
**Maiengasse 56**
**CH-4056 Basel (CH)**
Erfinder : **Wehrli, Rudolf, Dr.**
**Dianastrasse 2**
**CH-4310 Rheinfelden (CH)**

**EP 0 050 093 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Cyclopropankarbonsäure-α-allenyl-3-phenoxybenzylester, Verfahren zu ihrer Herstellung und ihre Verwendung in der Schädlingsbekämpfung. Die Cyclopropankarbonsäure-α-allenyl-3-phenoxybenzylester haben die Formel

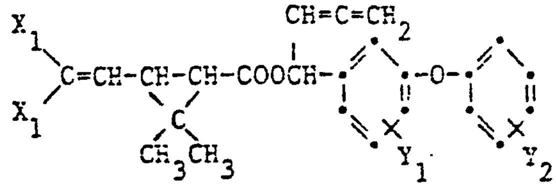

(I)

worin $X_1$ Halogen und $Y_1$ und $Y_2$ je Wasserstoff oder Halogen bedeuten.

Unter Halogen sind dabei Fluor, Chlor, Brom oder Jod, insbesondere oder Fluor, Chlor oder Brom, zu verstehen.

Bevorzugt sind Verbindungen der Formel I, worin $X_1$ Fluor oder Chlor, $Y_1$ Wasserstoff und $Y_2$ Wasserstoff, Fluor, Chlor oder Brom bedeuten.

Insbesondere bevorzugt sind Verbindungen der Formel I, worin $X_1$ Chlor, $Y_1$ Wasserstoff und $Y_2$ Wasserstoff, Fluor oder Chlor bedeuten.

Die Verbindungen der Formel I werden nach an sich bekannten Methoden, z. B. wie folgt hergestellt :

1)

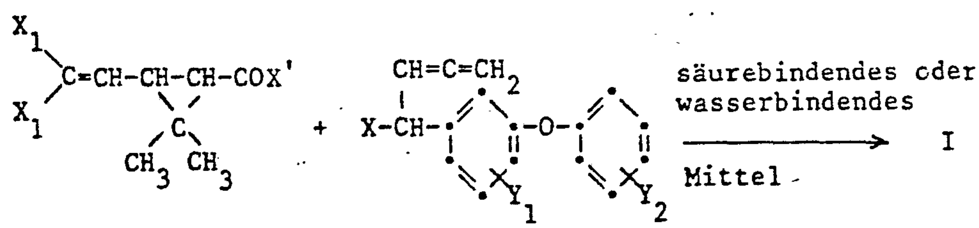


(II) (III)

2)

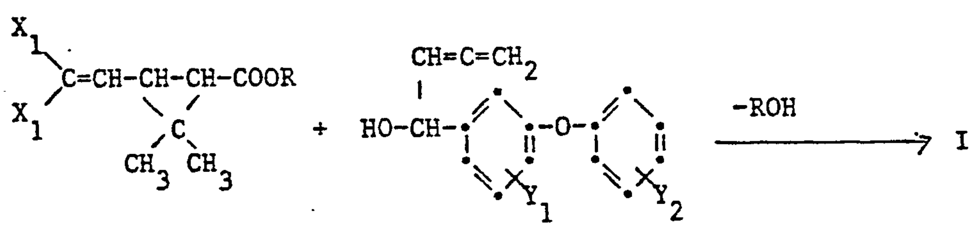


(IV) (V)

In den Formeln II bis V haben $X_1$, $Y_1$ und $Y_2$ die für die Formel I angegebene Bedeutung.

In den Formeln II und III steht eines der Symbole X und X' für eine Hydroxylgruppe und das andere für ein Halogenatom, insbesondere für Chlor oder Brom, und in der Formel IV steht R für $C_1$-$C_4$-Alkyl, insbesondere für Methyl oder Aethyl. Als säurebindende Mittel kommen insbesondere tertiäre Amine, wie Trialkylamine und Pyridin, ferner Hydroxide, Oxide, Carbonate und Bicarbonate von Alkali- und Erdalkalimetallen sowie Alkalimetallalkoholate, wie z. B. Kalium-t. butylat und Natriummethylat in Betracht. Als wasserbindendes Mittel kann z. B. Dicyclohexylcarbodiimid verwendet werden. Die Verfahren 1 und 2 werden bei einer Reaktionstemperatur zwischen – 10 und 120 °C, meist zwischen 20 und 80 °C, bei normalem oder erhöhtem Druck und vorzugsweise in einem Verdünnungsmittel eignen sich z. B. Aether und ätherartige Verbindungen, wie Diäthyläther, Dipropyläther, Dioxan, Dimethoxyäthan und Tetrahydrofuran ; Amide, wie N,N-dialkylierte Carbonsäureamide ; aliphatische, aromatische sowie halogenierte Kohlenwasserstoffe, insbesondere Benzol, Toluol, Xylole, Chloroform und Chlorbenzol ; Nitrile wie Acetonitril ; Dimethylsulfoxid und Ketone wie Aceton und Methyläthylketon.

Die Ausgangsstoffe der Formeln II und IV sind bekannt oder können analog bekannten Methoden hergestellt werden. Die Ausgangsstoffe der Formeln III sind neu. Sie werden analog Beispiel 1A hergestellt.

Die Verbindungen der Formel I liegen als Gemische von verschiedenen optisch aktiven Isomeren vor, wenn bei der Herstellung nicht einheitlich optisch aktive Ausgangsmaterialien verwendet werden. Die

verschiedenen Isomerengemische können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen Schädlingen an Tieren und Pflanzen.

Insbesondere eignen sich die Verbindungen der Formel I zur Bekämpfung von Insekten, phytopathogenen Milben und von Zecken, z. B. der Ordnung Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwoll- und Reiskulturen (z. B. Spodoptera littoralis, Heliothis virescens, Chilo suppresalis und Laodelphax) sowie Gemüse- und Obstkulturen (z. B. Leptinotarsa decemlineata, Myzus periscae, Laspeyresia pomonella und Adoxophyes reticulana.

Wirkstoffe der Formel I zeigen auch eine sehr günstige Wirkung gegen Fliegen, wie z. B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z. B. org. Phosphorverbindungen ; Nitrophenole und deren Derivate ; Formamidine ; Harnstoffe ; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u. a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfinyl)-propyl)-benzol.

Die Verbindungen der Formel I werden in unveränderter Form oder vorzugsweise zusammen mit den in der Formulierungstechnik üblichen Hilfsmitteln eingesetzt und werden daher z. B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle wie epoxidiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z. B. Bimsstein, Ziegelbruch, Sepiolit oder Bentonit, als nicht sorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen, die z. B. aus Kokosnuss- oder Talgöl gewonnen werden können. Ferner sind auch die Fettsäuremethyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch

den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z. B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxid-Adduktes in Frage.

Als nichtionische Tenside kommen in erster linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxid-Addukte, Tributylphenoxypolyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z. B. das Stearyltrimethylammoniumchlorid oder das Benzyl-di(2-chloräthyl)-äthylammoniumbromid.

Die in der Formulierungstechnik gebräuchlichen Tenside sind u. a. in folgender Publikation beschrieben :

« Mc Cutcheon's Detergents and Emulsifiers Annual » MC Publishing Corp., Ridgewood, New Jersey, 1979.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I (% = Gewichtsprozent)

| 1. Emulsions-Konzentrate | a) | b) | c) |
|---|---|---|---|
| Wirkstoff | 20 % | 40 % | 50 % |
| Ca-Dodecylbenzolsulfonat | 5 % | 8 % | 5,8 % |
| Ricinusöl-polyäthylenglykoläther (36 Mol AeO) | 5 % | — | — |
| Tributylphenoyl-polyäthylenglykoläther (30 Mol AeO) | — | 12 % | 4,2 % |
| Cyclohexanon | — | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80 % | 10 % | 5 % | 95 % |
| Aethylenglykolmonomethyläther | 20 % | — | — | — |
| Polyäthylenglykol MG 400 | — | 70 % | — | — |
| N-Methyl-2-pyrrolidon | — | 20 % | — | — |
| Epoxidiertes Kokosnussöl | — | — | 1 % | 5 % |
| Benzin (Siedegrenzen 160-190 °C | — | — | 94 % | — |

Die Lösungen sind zur Anwendung in Form kleinster Tropfen geeignet.

| 3. Granulate | a) | b) |
|---|---|---|
| Wirkstoff | 5 % | 10 % |
| Kaolin | 94 % | — |
| Hochdisperse Kieselsäure | 1 % | — |
| Attapulgit | — | 90 % |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2 % | 5 % |
| Hochdisperse Kieselsäure | 1 % | 5 % |
| Talkum | 97 % | — |
| Kaolin | — | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 5. Spritzpulver | a) | b) |
|---|---|---|
| Wirkstoff | 20 % | 60 % |
| Na-Ligninsulfonat | 5 % | 5 % |
| Na-Laurylsulfat | 3 % | — |
| Na-Diisobutylnaphthalinsulfonat | — | 6 % |
| Octylphenolpolyäthylenglykoläther (7-8 Mol AeO) | — | 2 % |
| Hochdisperse Kieselsäure | 5 % | 27 % |
| Kaolin | 67 % | — |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrat | |
|---|---|
| Wirkstoff | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5 % | 8 % |
| Talkum | 95 % | — |
| Kaolin | — | 92 % |

Man erhält anwendungsfertige Stäubemittel, indem der Wirkstoff mit dem Träger vermischt und auf einer geeigneten Mühle vermahlen wird.

| 8. Extruder Granulat | |
|---|---|
| Wirkstoff | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulat

| | |
|---|---|
| Wirkstoff | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1

A) Herstellung von α-Allenyl-3-phenoxybenzylalkohol

10 g α-Aethinyl-3-phenoxy-benzylalkohol werden zusammen mit 2,1 g Paraformaldehyd, 5,3 g Diisopropylamin, 0,215 g CuBr und 50 ml Dioxan während 2 Stunden unter Rückfluss gekocht. Man kühlt das Reaktionsgemisch auf 20 °C ab, giesst es auf 2 N HCl-Lösung und extrahiert mit Aether. Die organische Phase wird mit 10 %iger Kaliumkarbonat- und gesättigter Kochsalzlösung gewaschen, über Magnesiumsulfat getrocknet und eingeengt.

Nach dem Chromatographieren des Produkts an Kieselgel mit Hexan/Aether 7 : 3 als Eluiermittel erhält man die Verbindung der Formel

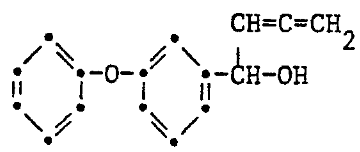

mit einem Brechungsindex von $n_D^{20°} = 1{,}594\,2$. Auf analoge Weise werden auch folgende Verbindungen hergestellt :

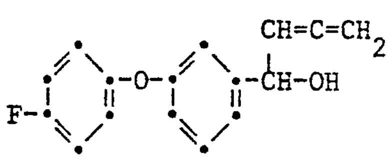

$n_D^{20} = 1{,}5765$

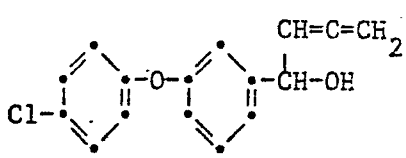

$n_D^{20°} = 1{,}5832$

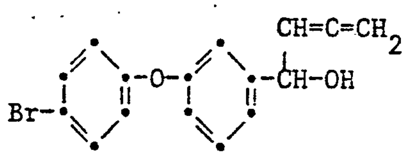

$n_D^{20°} = 1{,}5893$

B) Herstellung von α-Allenyl-3-phenoxybenzyl-2,2-dimethyl-3-(2,2-dichlorvinyl)-cyclopropan-1-carboxylat

5 g α-Allenyl-3-phenoxybenzylalkohol gelöst in 20 ml Toluol tropft man bei 0 °C zu einem Gemisch

von 4,8 g 2,2-Dimethyl-3-(2,2-dichlorvinyl)-cyclopropancarbonsäurechlorid, 2,2 ml Pyridin und 20 ml Toluol. Das Reaktionsgemisch wird 18 Stunden bei 20 °C gerührt, auf 2 N-Salzsäure gegossen und mit Toluol extrahiert. Die organische Phase wird mit 10 %iger Kaliumkarbonat- und gesättigter Kochsalzlösung gewaschen, über Magnesiumsulfat getrocknet und eingeengt.

Nach dem Chromatographieren des Rohproduktes über Kieselgel mit Aether/Hexan 1 : 10 als Eluiermittel erhält man die Verbindung der Formel

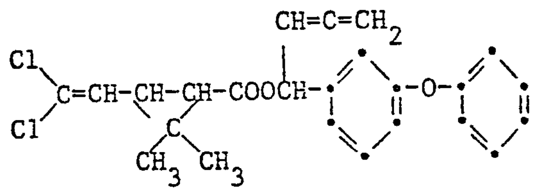

mit einem Brechungsindex von $n_D^{20°}$ = 1,575 7.

Auf analoge Weise werden auch folgende Verbindungen hergestellt :

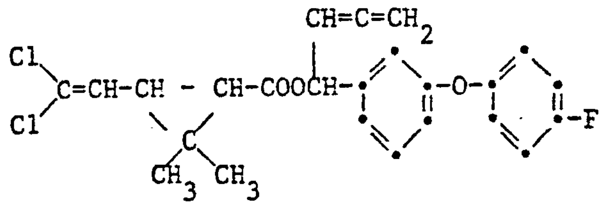

$n_D^{20°}$ = 1,5568

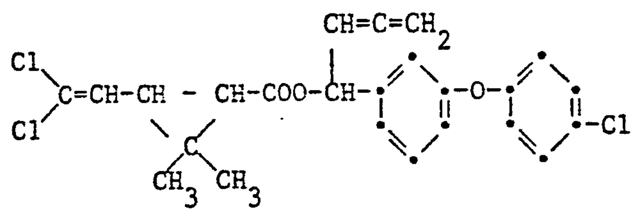

$n_D^{20°}$ = 1,5748

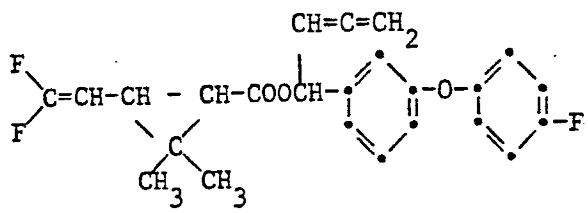

$n_D^{30°}$ = 1,5568

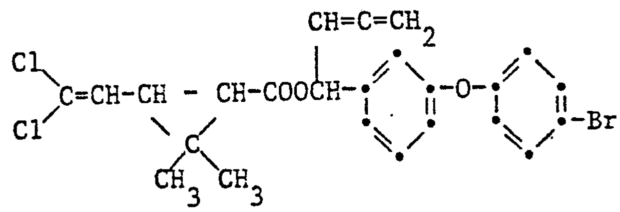

$n_D^{20°}$ =1,5938

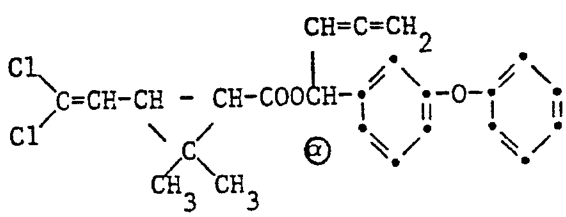

$n_D^{35°}$ = 1,5719

1 R-cis, α RS

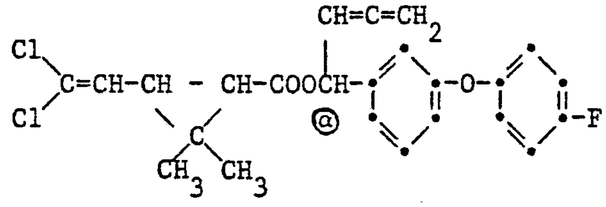

$n_D^{35°} = 1,5627$

1R-cis, α RS

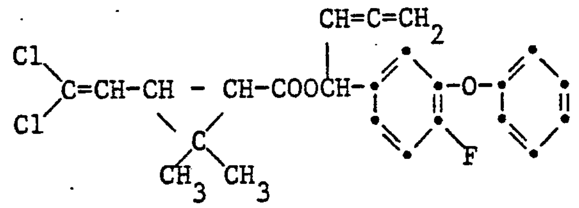

Beispiel 2

Insektizide Frassgift-Wirkung : Spodoptera littoralis, und Heliothis virescens

Baumwollpflanzen wurden mit einer Versuchslösung, enthaltend 50, 100, 200 oder 400 ppm der zu prüfenden Verbindung, besprüht.

Nach dem Antrocknen des Belages wurden die Pflanzen je mit Larven des Spezies Spodoptera littoralis (L3-Stadium) oder Heliothis virescens (L3) besetzt. Man verwendete pro Versuchsverbindung und pro Test-Spezies zwei Pflanzen. Die Auswertung der erzielten Abtötungsrate erfolgte nach 2, 4, 24 und 48 Stunden. Der Versuch wurde bei 28 °C und 60 % relativer Luftfeuchtigkeit durchgeführt.

Innerhalb der oben angegebenen Konzentrationsgrenzen zeigten Verbindungen gemäss dem Herstellungsbeispiel 1 Wirkung gegen Larven der Spezies Spodoptera littoralis und Heliothis virescens. (vgl. Tabelle)

Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit einer Versuchslösung, enthaldend 50, 100, 200, 400 und 800 ppm der zu prüfenden Verbindung, derart besprüht, dass kein Ablaufen der Spritzbrühe eintrat. Nach zwei und 7 Tagen wurden Larven, Adulte und Eier unter dem Binokular auf lebende und tote Individuen ausgewertet und das Ergebnis in Prozenten ausgedrückt. Während der « Haltezeit » standen die behandelten Pfanzen in Gewächshauskabinen bei 25 °C.

Innerhalb der oben angegebenen Konzentrationsgrenzen zeigten Verbindungen gemäss Beispiel 1 Wirkung gegen Adulte, Larven und Eier von Tetranychus urticae. (Vgl. Tabelle)

Beispiel 4

Frass- und Tiefenwirkung gegen Adoxophyes reticulana $L_3$ Larven

Pro Konzentration wurden 2 Apfelbäumchen (20 cm hoch) mit je 5 bis 8 Adoxophyes reticulana $L_3$ Larven infiziert. Diesen Larven wurde während 3 Tagen Gelegenheit gegeben, sich in ein Blatt einzurollen. Vor der Behandlung wurden die Blätter auf eingerollte Larven untersucht. Nicht oder ungegenügend eingerollte Larven wurden entfernt.

Drei Tage nach der Infestation wurden die Apfelbäumchen je mit 25 ml Versuchslösung, enthaltend 50 oder 100 ppm der zu prüfenden Verbindung, besprüht.

Die Auswertung auf lebende, bzw. tote Larven erfolgte 3 Tage nach der Behandlung. Innerhalb der oben angegebenen Konzentrationsgrenzen zeigten Verbindungen gemäss Beispiel 1 Wirkung gegen $L_3$-Larven von Adoxophyes reticulana (vgl. Tabelle).

Biologische Versuchsergebnisse

In der folgenden Tabelle sind Versuchsergebnisse auf der Basis der vorstehenden Beispiele

aufgeführt, und zwar mit folgendem Bewertungsindex in bezug auf die prozentuale Abtötung der Schädlinge :

A : 70-100 % Abtötung bei 50 ppm Wirkstoffkonzentration
B : 70-100 % Abtötung bei 100 ppm Wirkstoffkonzentration
C : 70-100 % Abtötung bei 200 ppm Wirkstoffkonzentration
D : 70-100 % Abtötung bei 400 ppm Wirkstoffkonzentration

Tabelle

| Verbindungen 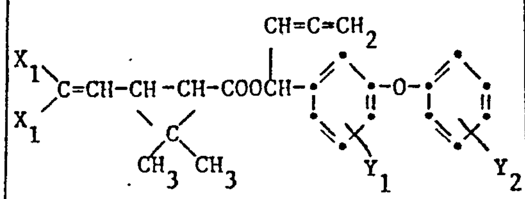 | | | Wirksamkeit | | | |
|---|---|---|---|---|---|---|
| $X_1$ | $Y_1$ | $Y_2$ | Spodoptera littoralis Larven | Heliothis virescens Larven | Tetranychus urticae Larven | Adoxophyes reticulana Larven |
| Cl | H | H | A | B | B | A |
| Cl | H | 4-F | B | B | B | B |
| Cl | H | 4-Cl | B | B | C | B |
| F | H | 4-F | C | D | D | A |

**Ansprüche**

1. Ein Cyclopropankarbonsäure-α-allenyl-3-phenoxybenzylester der Formel

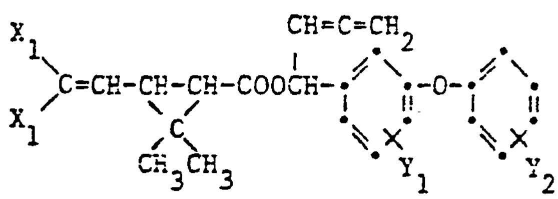

(I),

worin $X_1$ Halogen und $Y_1$ und $Y_2$ je Wasserstoff oder Halogen bedeuten.

2. Eine Verbindung gemäss Anspruch 1, worin $X_1$ Fluor oder Chlor, $Y_1$ Wasserstoff und $Y_2$ Wasserstoff, Fluor, Chlor oder Brom Bedeuten.

3. Eine Verbindung gemäss Anspruch 1, worin $X_1$ Chlor, $Y_1$ Wasserstoff und $Y_2$ Wasserstoff, Fluor oder Chlor bedeuten.

4. Die Verbindung gemäss Anspruch 3 der Formel

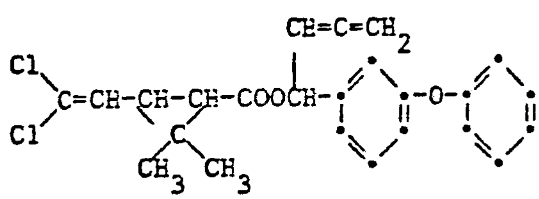

5. Die Verbindung gemäss Anspruch 3 der Formel

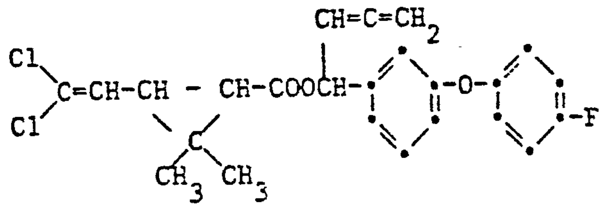

6. Die Verbindung gemäss Anspruch 3 der Formel

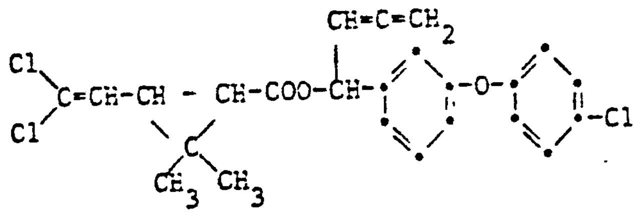

7. Eine Verbindung gemäss Anspruch 2 der Formel

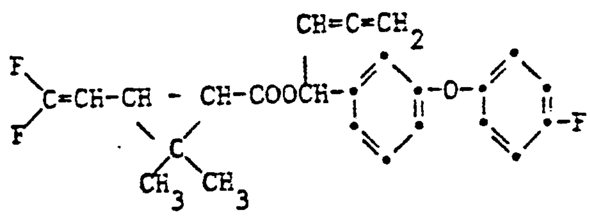

8. Eine Verbindung gemäss Anspruch 2 der Formel

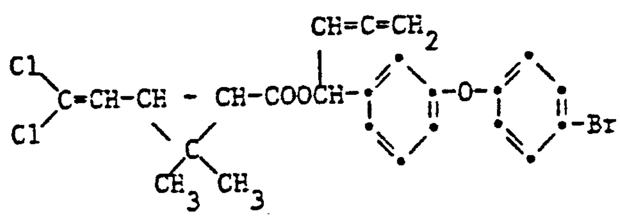

9. Eine Verbindung gemäss Anspruch 3 der Formel

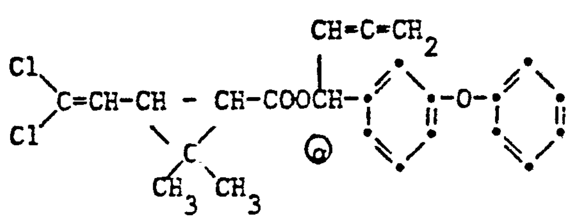

1 R-cis, α RS

10. Eine Verbindung gemäss Anspruch 3 der Formel

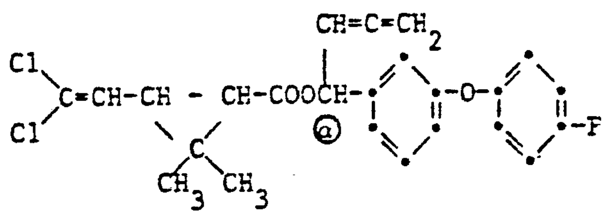

1 R-cis, α RS

11. Verfahren zur Herstellung von Verbindungen gemäss Anspruch 1, dadurch gekennzeichnet, dass man eine Verbindung der Formel

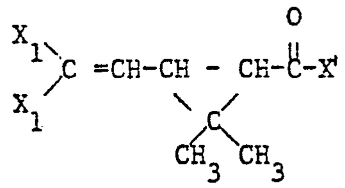

in Gegenwart eines säurebindenden Mittels mit einer Verbindung der Formel

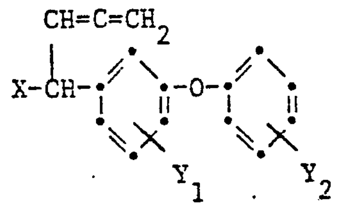

umsetzt, worin $X_1$, $Y_1$ und $Y_2$ die im Anspruch 1 angegebene Bedeutung haben und eines der Symbole X und X' für eine Hydroxylgruppe und das andere für ein Halogenatom steht.

12. Verwendung einer Verbindung gemäss Anspruch 1 zur Bekämpfung von verschiedenartigen Schädlingen an Tieren und Pflanzen.

13. Verwendung gemäss Anspruch 12 zur Bekämpfung von Insekten und Vertretern der Ordnung Akarina.

14. Ein Schädlingsbekämpfungsmittel, das als aktive Komponente eine Verbindung gemäss Anspruch 1 und geeignete Zusatzstoffe enthält.

15. Eine Verbindung der Formel

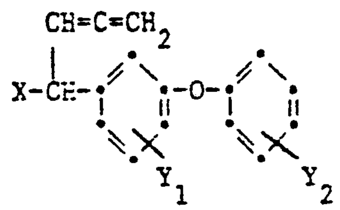

worin X Hydroxyl oder Halogen und $Y_1$ und $Y_2$ je Wasserstoff oder Halogen bedeuten.

**Claims**

1. A cyclopropanecarboxylic acid α-allenyl-3-phenoxybenzyl ester of the formula

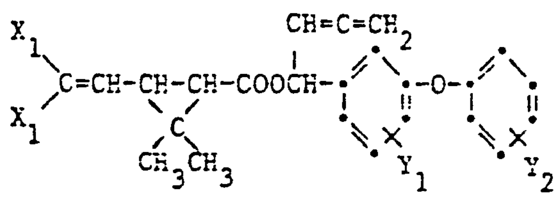

(I),

wherein $X_1$ is halogen and each of $Y_1$ and $Y_2$ is hydrogen or halogen.

2. A compound according to claim 1, wherein $X_1$ is fluorine or chlorine, $Y_1$ is hydrogen and $Y_2$ is hydrogen, fluorine, chlorine or bromine.

3. A compound according to claim 1, wherein $X_1$ is chlorine, $Y_1$ is hydrogen and $Y_2$ is hydrogen, fluorine or chlorine.

4. The compound according to claim 3 of the formula

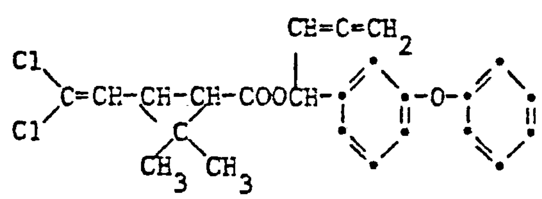

5. The compound according to claim 3 of the formula

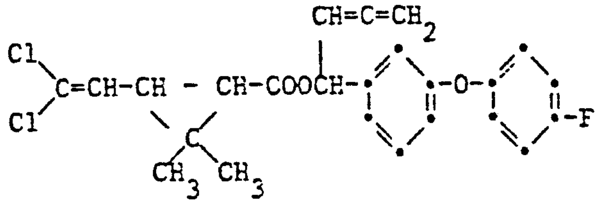

6. The compound according to claim 3 of the formula

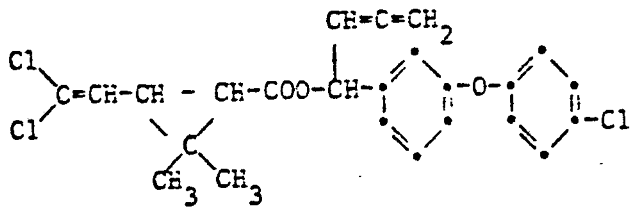

7. A compound according to claim 2 of the formula

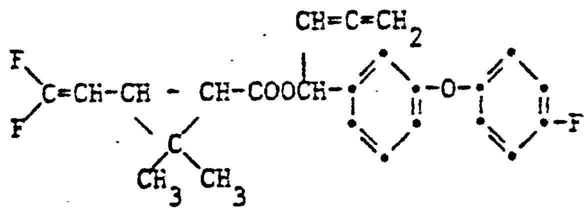

8. A compound according to claim 2 of the formula

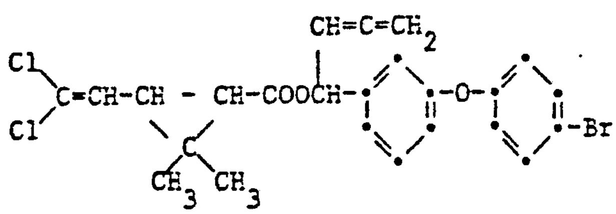

9. A compound according to claim 3 of the formula

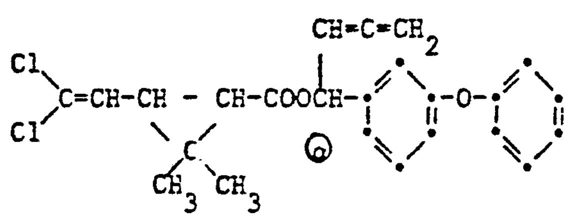

1 R-cis, α RS

10. A compound according to claim 3 of the formula

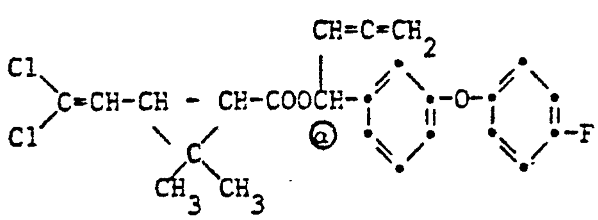

1 R-cis, α RS

11. A process for the production of a compound according to claim 1, which comprises reacting a compound of the formula

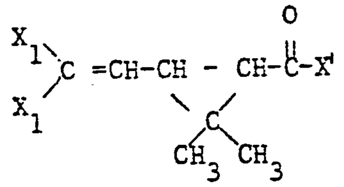

in the presence of an acid acceptor, with a compound of the formula

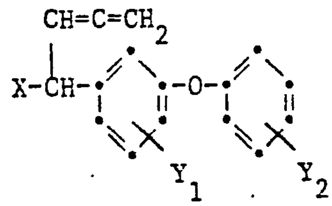

in which formulae $X_1$, $Y_1$ and $Y_2$ are as defined in claim 1 and one of X and X′ is a hydroxyl group and the other is a halogen atom.

12. A method of controlling a variety of pests of animals and plants at a locus, which method comprises applying to said locus a compound according to claim 1.

13. A method according to claim 12, wherein the pests to be controlled are insects and representatives of the order Acarina.

14. A pesticidal composition which comprises a pesticidally effective amount of a compound according to claim 1, together with suitable adjuvants.

15. A compound of the formula

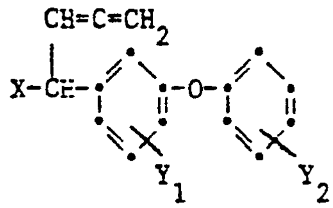

wherein X is hydroxyl or halogen and each of $Y_1$ and $Y_2$ is hydrogen or halogen.

**Revendications**

1. α-allényl-3-phénoxybenzylester de l'acide cyclopropanecarboxylique de formule

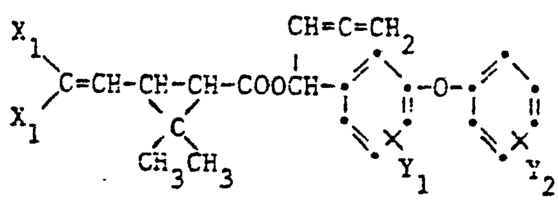

(I)

où $X_1$ représente un halogène et $Y_1$ et $Y_2$ représentent chacun un hydrogène ou un halogène.

2. Composé selon la revendication 1, où $X_1$ représente un fluor ou un chlore, $Y_1$ représente un hydrogène et $Y_2$ représente un hydrogène, un fluor, un chlore ou un brome.

3. Composé selon la revendication 1, où $X_1$ représente un chlore, $Y_1$ représente un hydrogène et $Y_2$ représente un hydrogène, un fluor ou un chlore.

4. Le composé selon la revendication 3 de formule

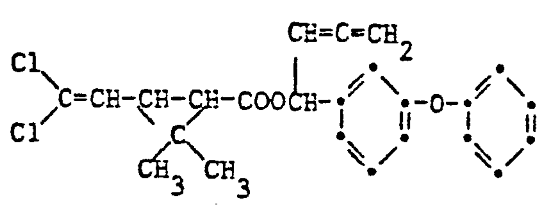

5. Le composé selon la revendication 3 de formule

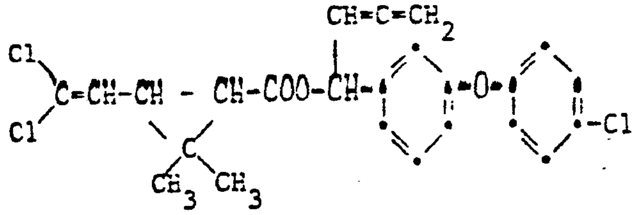

6. Le composé selon la revendication 3 de formule

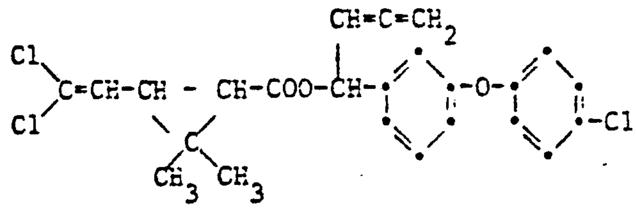

7. Composé selon la revendication 2 de formule

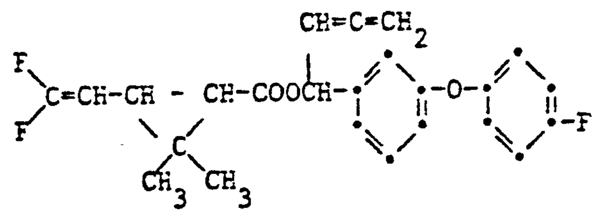

8. Composé selon la revendication 2 de formule

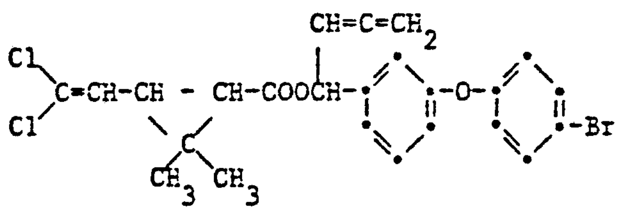

9. Composé selon la revendication 3 de formule

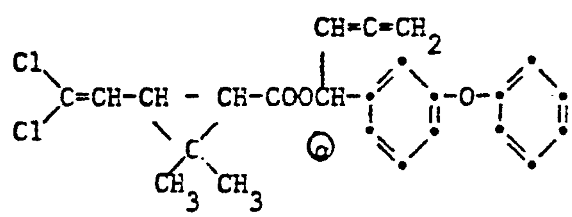

1 R-Cis, α RS

10. Composé selon la revendication 3 de formule

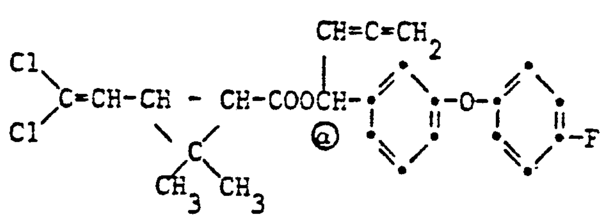

1 R-cis, α RS

11. Procédé de préparation de composés selon la revendication 1, caractérisé en ce qu'on fait réagir un composé de formule

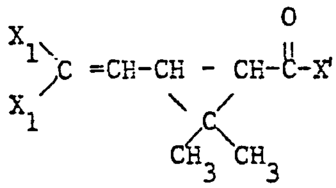

en présence d'un agent liant acide avec un composé de formule

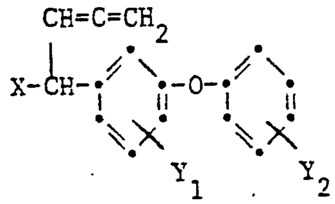

où $X_1$, $Y_1$ et $Y_2$ ont la signification donnée dans la revendication 1 et l'un des symboles X et X' représente un groupe hydroxyle et l'autre un atome d'halogène.

12. Application d'un composé selon la revendication 1 à la lutte contre différents parasites des animaux et des plantes.

13. Application selon la revendication 12 à la lutte contre les insectes et les représentants de l'ordre des acariens.

14. Agent de lutte antiparasitaire contenant comme composant actif un composé selon la revendication 1 et des additifs appropriés.

15. Composé de formule

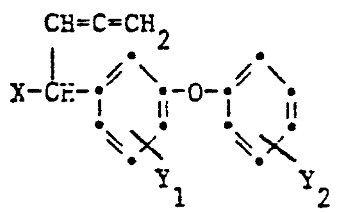

où X représente un hydroxyle ou un halogène et $Y_1$ et $Y_2$ représentent chacun un hydrogène ou un halogène.